Europäisches Patentamt

European Patent Office

Office européen des brevets

(11) Veröffentlichungsnummer: **0 441 256 A1**

## (12) EUROPÄISCHE PATENTANMELDUNG

(21) Anmeldenummer: **91101355.5**

(51) Int. Cl.⁵: **A61B 17/58**

(22) Anmeldetag: **01.02.91**

(30) Priorität: **08.02.90 DE 9001447 U**

(43) Veröffentlichungstag der Anmeldung:
**14.08.91 Patentblatt 91/33**

(84) Benannte Vertragsstaaten:
**AT BE CH DK ES FR GB IT LI NL SE**

(71) Anmelder: **Gebrüder Martin OHG**
**Ludwigstaler Strasse 132**
**W-7200 Tuttlingen(DE)**

(72) Erfinder: **Handte, Hermann**
**Ehrenbergstr. 39**
**W-7200 Tuttlingen(DE)**

(74) Vertreter: **Dipl.-Phys.Dr. Manitz**
**Dipl.-Ing.Dipl.-Wirtsch.-Ing. Finsterwald**
**Dipl.-Phys. Rotermund Dipl.-Chem.Dr. Heyn**
**B.Sc.(Phys.) Morgan**
**Robert-Koch-Strasse 1**
**W-8000 München 22(DE)**

(54) Knochennagel-Werkzeuganordnung.

(57) Eine Knochennagel-Werkzeuganordnung besteht aus einem Knochennagel (1) sowie einem mit diesem lösbar kuppelbaren Montagegriff (2) in dessen eine axiale Aufnahmeöffnung aufweisendes vorderes Ende der distale Endabschnitt des Knochennagels (1) einsetzbar ist und an dessen hinterem Endbereich eine für die Ausübung der Kräfte und Drehmomente ausgebildete Handhabe vorgesehen ist, wobei der Knochennagel (1) in die axiale Aufnahmeöffnung des Montagegriffes (2) derart eingesetzt und dort mittels eines Verriegelungsstiftes (32) gesichert ist, daß mittels des Montagegriffes (2) sowohl in beiden Axialrichtungen Kräfte als auch in beide Richtungen um die Nagelachse Drehmomente ausgeübt werden können. Die axiale Aufnahmeöffnung ist durch eine Axialbohrung (20) gebildet. Der Knochennagel (1) weist in seinem distalen Endabschnitt (10) eine Querbohrung (12) und der Montagegriff (2) im Bereich der Axialbohrung (20) den komplementär zur Querbohrung (12) ausgebildeten Verriegelungsstift (32) auf, wodurch bei im Montagegriff (2) verriegeltem Knochennagel (1) zwischen Verriegelungsstift (32) und dem Knochennagel-Endabschnitt (10) im Bereich der Querbohrung (12) eine Übertragung von axialen Kräften und Drehmomenten um die Nagelachse ermöglicht wird.

Fig. 2

EP 0 441 256 A1

## KNOCHENNAGEL-WERKZEUGANORDNUNG

Die Erfindung betrifft eine Knochennadel-Werkzeuganordnung bestehend aus einem Knochennagel sowie einem mit diesem lösbar kuppelbaren Montagegriff, in dessen eine axiale Aufnahmeöffnung aufweisendes vorderes Ende der distale Endabschnitt des Knochennagels einsetzbar ist und an dessen hinterem Endbereich eine für die Ausübung der Kräfte und Drehmomente ausgebildete Handhabe vorgesehen ist, wobei der Knochennagel in die axiale Aufnahmeöffnung des Montagegriffes derart eindesezt und dort mittels eines Verriegelungsstiftes gesichert ist, daß mittels des Montagegriffes sowohl in beiden Axialrichtungen Kräfte als auch in beiden Richtungen um die Nagelachse Drehmomente ausgeübt werden können.

Eine derartige Knochennagel-Werkzeuganordnung ist aus der DE-PS 23 41 439 bekannt. Dabei besitzt der Knochennagel an seinem distalen Endabschnitt einen plättchenförmig abgeflachten Ansatz, der sich in axialer Verlängerung des Nagels erstreckt. Der plättchenförmig abgeflachte Ansatz ist von einem Schlitz durchdrungen.

Die im Montagegriff vorgesehene axiale Aufnahmeöffnung für den plättchenförmig abgeflachten Ansatz ist in ihrem Querschnitt komplementär zum Querschnitt des plättchenförmig abgeflachten Ansatzes ausgebildet. Der Montagegriff ist im Bereich der Aufnahmeöffnung an einer Seite mit einer rechteckigen Öffnung versehen, die die Wandung der Aufnahmeöffnung in diesem Bereich durchdringt. Durch diese rechteckige Öffnung ist ein ebenfalls rechteckiger Haken gesteckt, der in den Schlitz des plättchenförmig abgeflachten Ansatzes eingreift.

Diese bekannte Knochennagel-Werkzeuganordnung erfordert ein aufwendiges Herstellungsverfahren für die dem plättchenförmig abgeflachten Ansatz komplementär ausgebildete Aufnahmeöffnung. Außerdem ist die Herstellung des plättchenförmig abgeflachten Ansatzes am Knochennagel kostspielig.

Darüber hinaus stellt der plättchenförmig abgeflachte Ansatz gegenüber dem Durchmesser des Knochennagels eine unerwünschte Materialverdünnung ausgerechnet in dem Bereich des Knochennagels dar, in dem Torsions-, Zug- oder Druckkräfte vom Montagegriff auf den Knochennagel übertragen werden müssen.

Es ist daher die Aufgabe der vorliegenden Erfindung eine Knochennagel-Werkzeuganorndung der eingangs genannten Gattung derart weiterzubilden, daß eine einfache und kostengünstige Herstellung ermöglicht wird, ohne dadurch die Funktionssicherheit beim Aufbringen von Zug-, Druck- oder Torsionskräften zu beeinträchtigen.

Diese Aufgabe wird bei der Knochennagel-Werkzeuganordnung der Erfindung dadurch gelöst, daß der Knochennagel in seinem distalen Endabschnitt eine Querbohrung und der Montagegriff im Bereich der Axialbohrung den komplementär zur Querbohrung ausgebildeten Verriegelungsstift aufweist, wodurch bei im Montagegriff ver riegeltem Knochennagel zwischen Verriegelungsstift und dem Knochennagel-Endabschnitt im Bereich der Querbohrung eine Übertragung von axialen Kräften und Drehmomenten um die Nagelachse ermöglicht wird.

Durch die Ausbildung der axialen Aufnahmeöffnung als Axialbohrung kann der Knochennagel selbst mit seinem axialen Endbereich in die Aufnahmeöffnung eingeführt werden, ohne daß es eines abgeflachten Ansatzes bedarf. Die Anordnung der Querbohrung im Knochennagel und des Verriegelungsstiftes im Montagegriff gewährleistet dabei sowohl die Übertragung von Torsionskräften als auch die Übertragung von Axialkräften zwischen Montagegriff und Knochennagel.

Durch die Ausbildung nach Anspruch 2 wird gewährleistet, daß auf den Montagegriff aufgebrachte Schlagkräfte überwiegend direkt vom Montagegriff auf den Knochennagel übertragen werden. Die Ausbildung nach Anspruch 3 entlastet dabei zusätzlich den Verriegelungsstift.

Die Ausbildung nach Anspruch 4 gestattet ein einfaches Zerlegen des Montagegriffs, wodurch eine Reinigung und insbesondere Sterilisation auf einfache Weise durchführbar ist. Die Ausbildung nach Anspruch 5 erlaubt ein schnelles Umsetzen des Montagegriffs von einem zu einem nächsten Knochennagel. Die Ausgestaltung nach Anspruch 6 zeichnet sich durch eine minimale Anzahl von Teilen aus, so daß der Montagegriff zu Zwecken der Reinigung und Sterilisation schnell zerlegt und ohne größeren Aufwand wieder zusammengesetzt werden kann. Außerdem wird durch diese Ausgestaltung eine kompakte Bauweise der Verriegelungsanordnung geschaffen.

Die nach Anspruch 7 vorgesehene Axialbohrung im Riegelbolzen gestatten den Durchblick auf den Verriegelungsstift und in die Queröffnung 35, so daß beim Einschieben eines Knochennagels dieser schnell und einfach so ausgerichtet werden kann, daß die im Knochennagel vorgesehene Querbohrung mit dem Verriegelungsstift fluchtet.

Auch die Ausbildung nach Anspruch 8 zeichnet sich durch eine Minimierung der Einzelteile aus, wodurch nicht nur das Zerlegen und der Zusammenbau des Montagegriffes erleichtert werden, sondern darüber hinaus die Gefahr minimiert wird, daß beim Reinigen und Sterilisieren Teile verlorengehen.

Die Erfindung wird nachfolgend anhand eines Beispiels unter Bezugnahme auf die Zeichnung erläutert, in dieser zeigt:

Figur 1    eine teilweise geschnittene Darstellung einer Knochennagel-Werkzeuganordnung der Erfindung,

Figur 2    eine vergrößerte Darstellung des geschnittenen Bereichs in Figur 1 und

Figur 3    eine Seitenansicht des Riegelbolzens in Richtung des Pfeiles III in Figur 2.

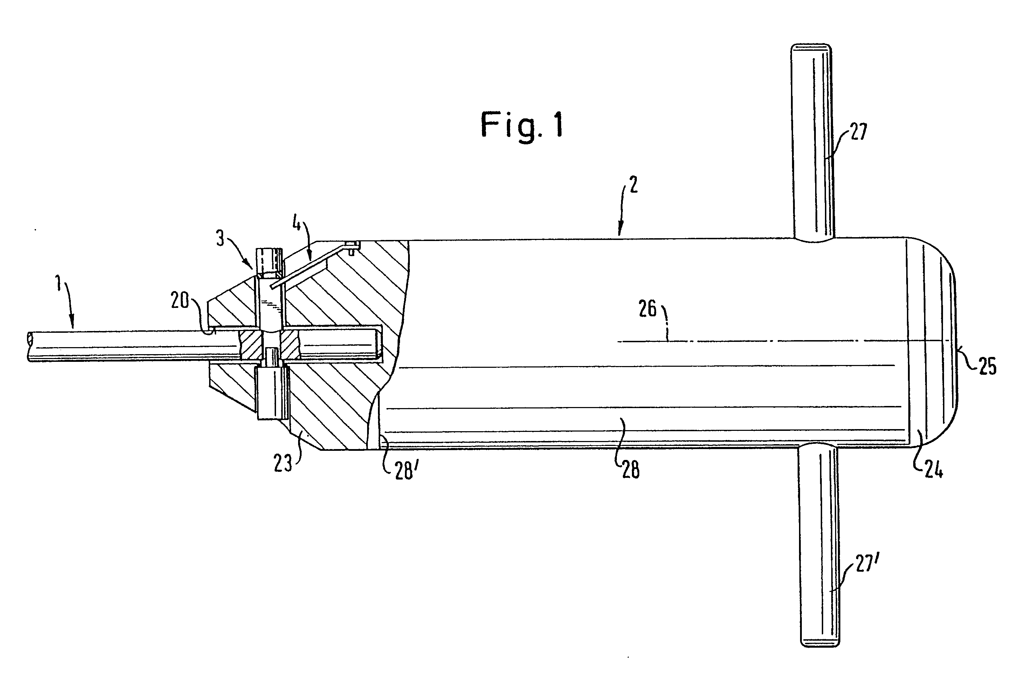

Figur 1 zeigt eine Knochennagel-Werkzeuganordnung nach der Erfindung, bei der ein Knochennagel 1 in eine Axialbohrung 20 eines Montagegriffes 2 eingesetzt und mittels eines von einer Feder 4 beaufschlagten Riegelbolzens 3 gehaltert ist.

Der Montagegriff 2 besitzt an seinem von der Axialbohrung 20 abgewandtem hinteren Endabschnitt 24 eine Schlagfläche 25, auf die mittels eines geeigneten Werkzeugs Schlagkräfte zum Eintreiben des Knochennagels in das Knochenmark aufgebracht werden können. Dem hinterem Ende benachbart erstrecken sich im wesentlichen rechtwinklig zur Längsachse 26 des Montagegriffs 2 ein erster und ein zweiter Hebelansatz (27, 27'). Die Hebelansätze 27, 27' münden an einander abgewandt gelegenen Seiten des Montagegriffs 2 und erstrecken sich entlang einer gemeinsamen, nicht gezeigten Achse. Die Hebelansätze 27, 27' dienen dazu, Torsinskräfte um die Längsachse 26 auf den Montagegriff 2 und damit auf den Knochennagel 1 aufzubringen.

Der Montagegriff 2 ist auf dem größten Teil seiner Umfangsfläche mit einer Oberflächenstruktur, wie beispielsweise einer Riffelung oder Rändelung versehen, wodurch ein Griffbereich 28 gebildet ist. Die Oberflächenstruktur ist in den Figuren nicht gezeigt, jedoch ist die vordere Grenze des Griffbereichs 28 durch die Linie 28' in der Nähe des vorderen Endabschnittes 23 des Montagegriffs 2 dargestellt. In die andere Richtung erstreckt sich der Griffbereich 28 bis in die Nähe der Schlagfläche 25, die nicht unbedingt eben ausgebildet sein muß, sondern auch kugelsegmentartig gewölbt sein kann. Eine derart gewölbte Schlagfläche 25 ist bei der Handhabung des Montagegriffs von Vorteil, da sie der Handinnenfläche der Bedienperson angepaßt ist.

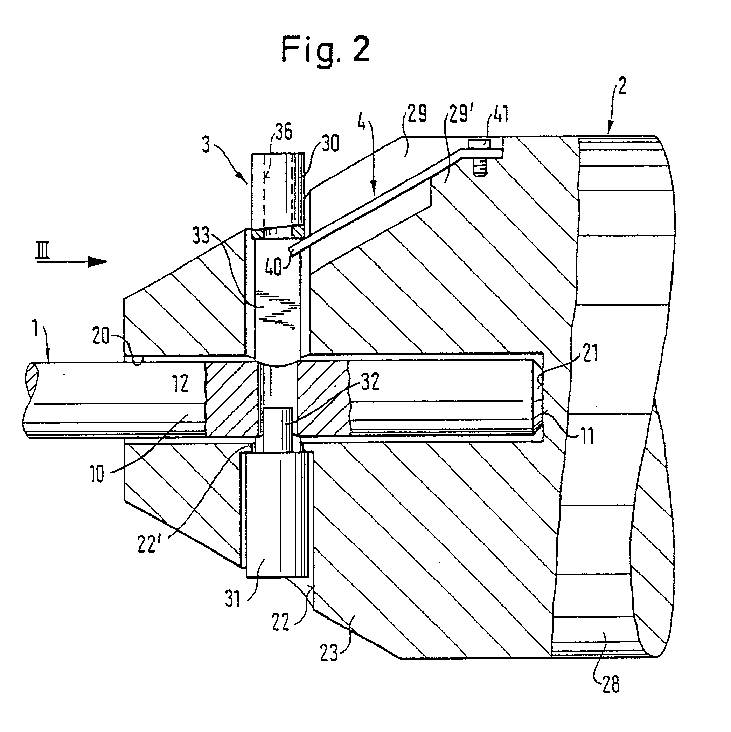

Figur 2 zeigt eine Ausgestaltung der Verriegelungsanordnung des Knochennagels 1 im Montagegriff 2. Der Montagegriff 2 besitzt dabei in seinem vorderen Endabschnitt 23 eine Querbohrung 22, die sich mit der Axialbohrung 20 kreuzt, wobei beide Bohrungen einander durchdringen.

Der vordere Endabschnitt 23 des Montgegriffs 2 ist kegelstumpfförmig ausgebildet, wobei die Querbohrung 22 im Bereich des Kegelstumpfs und vorzugsweise dort in der Nähe des größten·Umfangs gelegen ist.

Im Bereich des einen Austritts der Querbohrung 22 aus dem vorderen Endabschnitt 23 ist eine von der Querbohrung 22 in Richtung des Griffbereichs 28 verlaufende Nut 29 ausgebildet. Innerhalb dieser Nut ist am von der Bohrung 22 abgewandten Nutende eine Stufe 29' vorgesehen, auf der eine sich längs in der Nut erstreckende Blattfeder 4 abgestützt ist. Im Bereich der Stufe 29' ist die Blattfeder 4 auf bekannte Weise mit einer Schraube 41 am Montagegriff 2 befestigt. Die Feder 4 erstreckt sich dabei mit ihrem freien Endbereich 40 aus der Nut 29 in die Querbohrung 22 hinein. Die Feder 4 kann sich trotz ihrer Befestigung mit der Schraube 41 in der Nut in Radialrichtung des Montagegriffs 2 freifedernd bewegen.

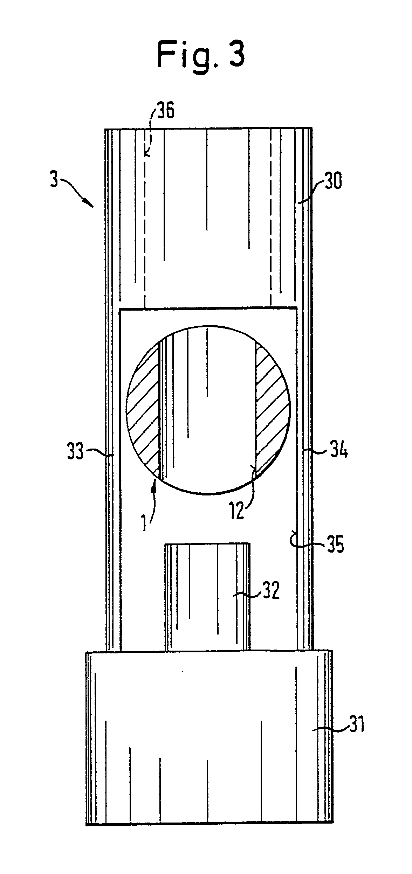

In die Querbohrung 22 ist ein Riegelbolzen 3 eingesetzt, der im folgenden in Zusammenhang mit Figur 3 näher beschrieben werden wird.

Der Riegelbolzen 3 ist ein im wesentlichen zylindrischer Körper mit einem ersten zylindrichen Abschnitt 30 sowie einem zweiten zylindrischen Abschnitt 31. Der zweite zylindrische Abschnitt 31 weist einen etwas größeren Durchmesser auf als der erste zylindriche Abschnitt 30. Der erste zylindriche Abschnitt 30 und der zweite zylindrische Abschnitt 31 sind durch seitliche Stege miteinander verbunden, die in ihrer äußeren Kontur die gleiche Krümmung aufweisen wie der erste zylindrische Abschnitt. Zwischen den Stegen 33, 34 und dem ersten zylindrischen Abschnitt 30 sowie dem zweiten zylindrichen Abschnitt 31 ist auf diese Weise eine im wesentlichen rechteckige Queröffnung 35 gebildet, die den Riegelbolzen 3 quer zu seiner Axialrichtung durchdringt. In diese Queröffnung 35 erstreckt sich koaxial mit dem zweiten zylindrischen Abschnitt und vorzugsweise in diesen als separates Teil eingesetzt ein zylindrischer Verriegelungsstift 32.

Der Durchmesser des Verriegelungsstiftes 32 ist deutlich kleiner als die Breite der Queröffnung 35. Der Durchmesser des Verriegelungsstiftes 32 beträgt dabei etwa 30 % bis 50 %, vorzugweise 40 % der Breite der Queröffnung 35.

Der Verriegelungsstift 32 erstreckt sich in Axialrichtung nur zum Teil in die Queröffnung 35. Diese Axialerstreckung des Verriegelungsstiftes 32 beträgt etwa 20 % bis 40 %, vorzugsweise 25 % der Längserstreckung der Queröffnung, wobei allerdings die Erstreckung des Verriegelungsstiftes 32 in die Queröffnung 35 hinein nur so groß sein darf, daß der verbleibende frei durchgängige Bereich der Queröffnung 35 in Längsrichtung mindestens dem Maß der Breite der Queröffnung entspricht. Hierdurch wird gewährleistet, daß ein kreisrunder Knochennagel, der in seinem Durchmesser der Breite der Queröffnung angepaßt ist, auch in Längsrichtung der Queröffnung durch diese hindurchgesteckt werden kann.

In Figur 3 ist ein Knochennagel 1 im Bereich seiner Querbohrung 12 geschnitten dargestellt, wobei der Knochennagel die Queröffnung 35 durchdringt. Die Querbohrung 12 des Knochennagels 1 ist dabei koaxial zum Verriegelungsstift 32 ausgerichtet, so daß der Verriegelungsstift 32 in die Querbohrung 12 des Knochennagels 1 eindringen kann. Dementsprechend sind die Durchmesser des Verriegelungsstiftes 32 und der Querbohrung 12 des Knochennagels 1 aufeinander abgestimmt. Die in die Queröffnung 35 hineinragende Länge des Verriegelungsstiftes sollte dabei derart bemessen sein, daß sie etwa 40 % bis 60 % des Knochennagel-Durchmessers ausmacht, vorzugsweise dem Radius des Knochennagels entspricht, wodurch bei kompakter Bauweise des Riegelbolzens 3 eine optimale Kraftübertragung zwischen Verriegelungsstift 32 und Knochennagel 1 gewährleistet ist.

Der Riegelbolzen 3 weist in seinem ersten zylindrischen Abschnitt 30 eine Axialbohrung 36 auf, die einen axialen Einblick auf den Verriegelungsstift 32 bzw. auf einen in die Queröffnung 35 eingesteckten Knochennagel zuläßt. Auf diese Weise ist ein schnelles und einfaches Ausrichten der Querbohrung 12 des Knochennagels koaxial zum Verriegelungsstift 32 möglich.

Wie in Figur 2 zu sehen ist, ist die Querbohrung 22 mit einer Ringschulter 22' versehen, gegen die der in seinem Durchmesser größere zweite zylindrische Abschnitt 31 des Riegelbolzens 3 anliegt.

Bei in die Querbohrung 24 eingesetztem Riegelbolzen 3 untergreift der in die Querbohrung 22 eindringende Endabschnitt 40 der Blattfeder 4 den ersten zylindrischen Abschnitt 30 des Riegelbolzens 3 im Bereich seiner Queröffnung 35. Auf diese Weise wird der Riegelbolzen 3 zwischen der Ringschulter 22' und der Feder 4 eingespannt und kann unter Auslenkung der Feder 4 von der Ringschulter 22' weggedrückt werden, wobei der Verriegelungsstift 32 aus der Axialbohrung 20 des Montagegriffes austritt und der freie Bereich der Queröffnung 35 mit der Axialbohrung 20 des Montagegriffs 2 fluchtet und den Durchtritt des Endabschnitts 10 eines Knochennagels 1 ermöglicht. Um den Knochennagel 1 in der Axialbohrung 20 sicher und mit wenig radialem Spiel führen zu können. sollte die Breite der Queröffnung 35 dem Durchmesser der Axialbohrung 20 angepaßt sein.

Zwischen dem Verriegelungsstift 32 und der Innenwandung der Querbohrung 12 des Knochennagels 1 sollte ein geringes radiales Spiel vorhanden sein, das bei Anlage der distalen Stirnfläche 11 des Knochennagels 1 an der Grundfläche 21 der als Sackbohrung ausgeführten Axialbohrung 20 einen Kontakt des Verriegelungsstiftes 32 mit der Innenwandung der Bohrung 12 des Knochennagels 1 verhindert, so daß über dem Montagegriff 2 auf dem Knochennagel 1 aufgebrachte axiale Schlagkräfte nur über die Berührungsfläche zwischen der Grundfläche 21 und der distalen Stirnfläche 11 übertragen werden.

Gleichzeitg sollte das Spiel zwischen dem Verriegelungsstift 32 und der Innenwandung der Querbohrung 12 des Knochennagels 1 jedoch nicht so groß sein, daß es beim Aufbringen von Torsionskräften oder Zugkräften auf den Montagegriff ein gefühlvolles Arbeiten nicht mehr zuläßt.

Bezugszeichenliste

| | |
|---|---|
| 1 | Knochennagel |
| 2 | Montagegriff |
| 3 | Riegelbolzen |
| 4 | Feder |
| 10 | Distaler Endabschnitt |
| 11 | Distale Stirnfläche |
| 12 | Querbohrung |
| 20 | Axialbohrung |
| 21 | Grundfläche |
| 22 | Querbohrung |
| 22' | Ringschulter |
| 23 | Vorderer Endabschnitt |
| 24 | Hinterer Endabschnitt |
| 25 | Schlagfläche |
| 26 | Längsachse |
| 27, 27' | Hebelansätze |
| 28 | Griffbereich |
| 28' | Vordere Begrenzung |
| 29 | Nut |
| 30 | Erster zylindricher Abschnitt |
| 31 | Zweiter zglindrischer Abschnitt |
| 32 | Verriegelungsstift |
| 33 | Steg |
| 34 | Steg |
| 35 | Queröffnung |
| 36 | Axialbohrung |
| 40 | Freier Endbereich |
| 41 | Schraube |

**Patentansprüche**

1.  Knochennagel-Werkzeuganordnung bestehend aus einem Knochennagel sowie einem mit diesem lösbar kuppelbaren Montagegriff, in dessen eine axiale Aufnahmeöffnung aufweisendes vorderes Ende der distale Endabschnitt des Knochennagels einsetzbar ist und an dessen hinterem Endbereich eine für die Ausübung der Kräfte und Drehmomente ausgebildete Handhabe vorgesehen ist, wobei der Knochennagel in die axiale Aufnahmeöffnung des Montagegriffes derart eingesetzt und dort mittels eines Verriegelungsstiftes gesichert ist, daß mittels des Montagegriffes sowohl in beiden Axialrichtungen Kräfte als auch in beide Richtungen um die Nagelachse Drehmomente ausgeübt werden können, dadurch **gekennzeichnet**, daß die axiale Aufnahmeöffnung durch eine Axialbohrung (20) gebildet ist, daß der Knochennagel (1) in seinem distalen Endabschnitt (10)eine Querbohrung (12) und der Montagegriff (2) im Bereich der Axialbohrung (20) den komplementär zur Querbohrung (12) ausgebildeten Verriegelungsstift (32) aufweist, wodurch bei im Montagegriff (2) verriegeltem Knochennagel (1) zwischen Verriegelungsstift (32) und dem Knochennagel-Endabschnitt (10) im Bereich der Querbohrung (12) eine Übertragung von axialen Kräften und Drehmomenten um die Nagelachse ermöglicht wird.

2. Knochennagel-Werkzeuganordnung nach Anspruch 1, dadurch **gekennzeichnet**, daß die Stirnfläche (11) am distalen Endabschnitt (10) des Knochennagels (1) als im wesentlichen kreisförmige Schlagfläche ausgebildet ist, die an der Grundfläche (21) der als Sackloch ausgebildeten Axialbohrung (20) des Montagegriffes (2) anliegt wenn der Verriegelungsstift (32) in die Querbohrung (12) eingreift.

3. Knochennagel-Werkzeuganordnung nach Anspruch 2, dadurch **gekennzeichnet**, daß die Querbohrung (12) im Durchmesser um soviel größer ist als der Verriegelungsstift (32), daß die Bohrungswandung in dem von der distalen Stirnfläche (11) abgewandten Bereich bei Anlage der distalen Stirnfläche (11) des Knochennagels (1) an der Grundfläche (21) der Axialbohrung (20) bezüglich des Verriegelungsstiftes (32) ein geringes radiales Spiel aufweist.

4. Knochennagel-Werkzeuganordnung nach einem der vorhergehenden Ansprüche, dadurch **gekennzeichnet**, daß im vorderen Endabschnitt (23) des Montagegriffes (2) eine Querbohrung (22) vorgesehen ist, die von der Axialbohrung (20) durchdrungen ist und daß in die Querbohrung (22) ein Riegelbolzen (3) eingesetzt ist, der den Verriegelungsstift (32) trägt.

5. Knochennagel-Werkzeuganordnung nach Anspruch 4, dadurch **gekennzeichnet**,

daß der Riegelbolzen (3) gegen die Kraft einer Feder (4) axial bewegbar ist, wobei der Verriegelungsstift (32) unter Einfluß der Federwirkung in seine Verriegelungsstellung bewegt wird.

6. Knochennagel-Werkzeuganordnung nach Anspruch 4 oder 5, dadurch **gekennzeichnet**, daß der Riegelbolzen (3) einen ersten zylindrischen Abschnitt (30), einen zweiten zylindrischen Abschnitt (31) sowie zwei diese Abschnitte verbindende seitliche Stege (33, 34) aufweist, wodurch eine Queröffnung (35) gebildet ist, in die der Verriegelungsstift (32) hineinragt und die zum Durchtritt des distalen Endabschnittes (10) des Knochennagels (1) ausgebildet ist.

7. Knochennagel-Werkzeuganordnung nach Anspruch 6, dadurch **gekennzeichnet**, daß der nicht mit dem Verriegelungsstift (32) versehene erste zylindrische Abschnitt (30) eine Axialbohrung (36) aufweist.

8. Knochennagel-Werkzeuganordnung nach einem der vorhergehenden Ansprüche dadurch **gekennzeichnet**, daß die Feder (4) eine am Montagegriff (2) lösbar befestigte Blattfeder ist, die mit ihrem freien Endbereich (40) in die Queröffnung (35) des Riegelbolzens (3) eingreift und sich am ersten zylindrischen Abschnitt (30) abstützt.

Fig.1

# Fig. 2

# Fig. 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | US-A-3 974 868   (DERBYSHIRE) <br> * Zusammenfassung; Spalte 1, Zeilen 11-29,46-60; Spalte 3, Zeilen 10-24; Abbildungen 6,7 * <br> – – – | 1,4,5,8 | A 61 B 17/58 |
| Y | EP-A-0 194 014   (THACKRAY) <br> * Seite 10, Zeilen 6-23; Abbildung 3 * <br> – – – | 1,4,5,8 | |
| A | EP-A-0 094 489   (HOWMEDICA) <br> * Seite 10, Zeilen 12-23; Seite 13, Zeile 20 - Seite 14, Zeile 24; Abbildungen 2,7,8 * <br> – – – – – | 1 | |
| | | | **RECHERCHIERTE SACHGEBIETE (Int. Cl.5)** <br> A 61 B <br> A 61 F <br> B 25 G |

Der vorliegende Recherchenbericht wurde für alle Patentansprüche erstellt

| Recherchenort | Abschlußdatum der Recherche | Prüfer |
|---|---|---|
| Den Haag | 10 April 91 | GIMENEZ BURGOS R. |